# EUROPEAN PATENT APPLICATION

(11) **EP 4 825 959 A1**
(43) Date of publication of application: **21.10.2026**
(21) Application number: 25738919.7
(22) Date of filing: 07.01.2025
(51) Int. Cl.: C12N 1/20, A01N 63/22, A01P 21/00, C12R 1/07

(54) **NOVEL BACILLUS AMYLOLIQUEFACIENS STRAIN HAVING ACTIVITY OF PROMOTING PLANT RESISTANCE AGAINST COLD STRESS AND USES THEREOF**

(30) Priority: 08.01.2024 KR 20240003016
(71) Applicant: CJ Cheiljedang Corporation, Seoul 04560 (KR)
(72) Inventor: OH, Young Joo, Seoul 04560 (KR); KANG, Yu Na, Seoul 04560 (KR); LEE, Mi Rong, Seoul 04560 (KR); SON, Ju Yeon, Seoul 04560 (KR); YUN, Yeo Hong, Seoul 04560 (KR)
(74) Representative: Meissner Bolte Partnerschaft mbB
(86) International application number: PCT/KR2025/000330
(87) International publication number: WO 2025/150845

(57) **Abstract**

The present disclosure relates to a novel *Bacillus amyloliquefaciens* strain, which enhances the stress resistance of plants and promotes the growth of plants. The *Bacillus amyloliquefaciens* N484 CP02-1006 strain, the *Bacillus amyloliquefaciens* N485 CP02-1007 strain, or a culture thereof of the present disclosure can effectively mitigate the reduction in fresh weight, leaf length, and leaf width of plants caused by cold stress.

## Description

### [Technical Field]

### [Cross reference to related application]

The present disclosure claims the benefit of priority based on Korean Patent Application No. 10-2024-0003016 filed on January 8, 2024, the entire contents of which are incorporated as part of this specification.

### [Technical Field]

The present disclosure relates to a novel *Bacillus amyloliquefaciens* strain having activity to enhance cold stress resistance of plants and the use thereof.

### [Background Art]

Plants have a significantly less ability to avoid various environmental stresses encountered during their survival process compared to animals, and therefore, environmental stresses such as low temperature, high temperature, salinity, or dryness have a significant impact on plant survival, growth, development, and yield. These environmental stresses are critical factors that significantly reduce crop production in farms, and biotic stresses such as the outbreak of plant diseases can also have a significant impact on crop cultivation.

Among these, temperature is one of the most important factors that can affect the survival of all plants and serves as a major driving force in plant growth and development. Cold stress is one of the major factors that restrict plant growth and development, and can inhibit normal plant growth by resulting in a reduction in photosynthetic rates, nutrient imbalances, and disruptions in secondary metabolic processes and hormones, thereby having a direct effect on plant productivity. Accordingly, it is necessary to develop products that minimize cold injury in plants caused by sudden temperature drops.

Meanwhile, with recent rising social concern over the adverse effects of agrochemicals, there is an increasing demand for environmentally friendly products that can replace chemical fertilizers and pesticides. Therefore, it is necessary to develop environmentally friendly products that can promote plant production by enhancing cold stress resistance of plants.

Korean Laid-Open Patent Application No. 10-2023-0109295 discloses the *Streptomyces anandii* J6 strain having properties that confer environmental stress resistance to plants, and discloses that this strain confers resistance to high temperature, dryness, and low temperature stresses to turfgrass. Korean Patent No. 10-2411304 discloses the *Bacillus zanthoxyli* HS1 strain having an excellent effect of enhancing resistance of a plant against various stresses. Korean Patent No. 10-2125456 discloses the *Bacillus siamensis* H30-3 strain, which promotes the resistance of plant against biotic or abiotic stress.

### [Disclosure]

### [Technical Problem]

It is an object of the present disclosure to provide a novel strain that enhances plant stress resistance and promotes plant growth.

It is another object of the present disclosure to provide a composition for enhancing plant stress resistance.

It is another object of the present disclosure to provide a composition for promoting plant growth.

It is another object of the present disclosure to provide a method for enhancing plant stress resistance.

It is another object of the present disclosure to provide a method for promoting plant growth.

### [Technical Solution]

Each description and embodiment disclosed in the present disclosure may also be applied to each different description and embodiment. That is, all combinations of the various elements disclosed in the present disclosure fall within the scope of the present disclosure. In addition, it cannot be seen that the scope of the present disclosure is limited by the specific descriptions described below. In addition, a person skilled in the art can recognize or ascertain, using only routine experiment, numerous equivalents to the specific embodiments of the present disclosure described in the present disclosure. In addition, such equivalents are intended to be included in the present disclosure.

In order to achieve the above objects, one aspect of the present disclosure provides a *Bacillus amyloliquefaciens* N484 CP02-1006 strain deposited under Accession No. KCCM13395P.

In addition, another aspect of the present disclosure provides a *Bacillus amyloliquefaciens* N485 CP02-1007 strain deposited under Accession No. KCCM13396P.

In addition, another aspect of the present disclosure provides a composition for enhancing plant stress resistance, comprising, as an active ingredient, one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

In addition, another aspect of the present disclosure provides a composition for promoting plant growth, comprising, as an active ingredient, one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

In addition, another aspect of the present disclosure provides a method for enhancing plant stress resistance, comprising treating a plant, an area surrounding the plant, or both, with one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

In addition, another aspect of the present disclosure provides a method for promoting plant growth, comprising treating a plant, an area surrounding the plant, or both, with one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

Hereinafter, the present disclosure will be described in detail.

One aspect of the present disclosure provides a *Bacillus amyloliquefaciens* N484 CP02-1006 strain deposited under Accession No. KCCM13395P.

In addition, another aspect of the present disclosure provides a *Bacillus amyloliquefaciens* N485 CP02-1007 strain deposited under Accession No. KCCM13396P.

The *Bacillus amyloliquefaciens* N484 CP02-1006 strain deposited under Accession No. KCCM13395P was deposited internationally with the Korean Culture Center of Microorganisms, which is an international depository institution under the Budapest Treaty, and was assigned the Accession No. KCCM13395P on September 21, 2023. In this specification, the strain may also be used interchangeably as *Bacillus amyloliquefaciens* N484 CP02-1006 strain, N484 CP02-1006 strain, N484 strain, or CP02-1006 strain.

The *Bacillus amyloliquefaciens* N484 CP02-1006 strain deposited under Accession No. KCCM13395P may comprise a 16S rRNA gene nucleotide sequence having about 95% or more, about 96% or more, about 97% or more, about 98% or more, about 99% or more, about 99.5% or more, or about 99.9% or more sequence identity to the nucleotide sequence of SEQ ID NO: 1. Specifically, the N484 CP02-1006 strain may comprise a 16S rRNA gene nucleotide sequence containing the nucleotide sequence of SEQ ID NO: 1.

The *Bacillus amyloliquefaciens* N485 CP02-1007 strain deposited under Accession No. KCCM13396P was deposited internationally with the Korean Culture Center of Microorganisms, which is an international depository institution under the Budapest Treaty, and was assigned the Accession No. KCCM13396P on September 21, 2023. In this specification, the strain may also be used interchangeably as *Bacillus amyloliquefaciens* N485 CP02-1007 strain, N485 CP02-1007 strain, N485 strain, or CP02-1007 strain.

The *Bacillus amyloliquefaciens* N485 CP02-1007 strain deposited under Accession No. KCCM13396P may comprise a 16S rRNA gene nucleotide sequence having about 95% or more, about 96% or more, about 97% or more, about 98% or more, about 99% or more, about 99.5% or more, or about 99.9% or more sequence identity to the nucleotide sequence of SEQ ID NO: 2. Specifically, the N485 CP02-1007 strain may comprise a 16S rRNA gene nucleotide sequence containing the nucleotide sequence of SEQ ID NO: 2.

In one embodiment, the N484 CP02-1006 strain and/or the N485 CP02-1007 strain may enhance plant stress resistance and/or promote plant growth.

In one embodiment, the stress may be cold stress. The cold stress causes a reduction in photosynthetic rates, nutrient imbalances, and disruptions in secondary metabolic processes and hormones in plants, thereby inhibiting normal plant growth.

In the present disclosure, the cold stress may be a temperature condition of -20 °C to 20 °C. For example, the cold stress may be a temperature condition of -20 °C to -12 °C, -20 °C to -4 °C, -20 °C to 8 °C, -20 °C to 12 °C, -12 °C to -4 °C, -12 °C to 4 °C, -12 °C to 12 °C, -12 °C to 20 °C, -4 °C to 4 °C, -4 °C to 12 °C, -4 °C to 20 °C, 4 °C to 12 °C, 4 °C to 20 °C, or 12 °C to 20 °C, but is not limited thereto.

In the present disclosure, the "plant" includes organisms belonging to the Kingdom Plantae that perform photosynthesis, and is not taxonomically limited to any specific plant.

In the present disclosure, the "plant" may be one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

The "Cucurbitaceae" plants may include, for example, cucumber, oriental melon, watermelon, cantaloupe, honeydew, melon, pumpkin, zucchini, and squash, and specifically, the Cucurbitaceae plants may be cucumber, oriental melon, watermelon, or melon, but are not limited thereto.

The "Brassicaceae" plants may include, for example, Chinese cabbage, arugula, broccoli, bok choy, Brussels sprouts, cabbage, cauliflower, canola, collard greens, daikon, garden cress, horseradish, kale, mustard, radish, rapeseed, rutabaga, turnip, wasabi, watercress, and *Arabidopsis thaliana*, and specifically, the Brassicaceae plants may be, but are not limited to, Chinese cabbage, radish, mustard, rapeseed, or *Arabidopsis thaliana*.

The "Amaranthaceae" plants may include, but are not limited to, for example, chard, spinach, sugar beet, and quinoa.

The "Amaryllidaceae" plants may include, but are not limited to, for example, scallion, onion, garlic, chive, leek, and shallot.

The "Apiaceae" plants may include, but are not limited to, for example, anise, caraway, carrot, celery, chervil, coriander, cumin, dill, fennel, parsley, and parsnip.

The "Asparagaceae" plants may include, but are not limited to, for example, agave and asparagus.

The "Asteraceae" plants may include, but are not limited to, for example, artichoke, aster, chamomile, chicory, chrysanthemum, dahlia, daisy, echinacea, goldenrod, guayule, lettuce, marigold, safflower, sunflower, and zinnia.

The "Bromeliaceae" plants may include, but are not limited to, for example, pineapple.

The "Convolvulaceae" plants may include, but are not limited to, for example, morning glory, and sweet potato.

The "Fabaceae" plants may include, but are not limited to, for example, alfalfa, beans, carob, clover, guar, lentils, mesquite, peas, peanuts, soybeans, tamarind, tragacanth, and vetch.

The "Malvaceae" plants may include, but are not limited to, for example, cacao, cotton, durian, hibiscus, kenaf, cola, and okra.

The "Musaceae" plants may include, but are not limited to, for example, banana and plantain.

The "Poaceae" plants may include, but are not limited to, for example, bamboo, barley, maize, fonio, millet, oats, ornamental grasses, rice, rye, sorghum, sugar cane, triticale, wheat, and turfgrasses such as bahiagrass, bermudagrass, bluegrass, buffalograss, centipedegrass, fescue, or zoysia.

The "Polygonaceae" plants may include, but are not limited to, for example, buckwheat.

The "Rosaceae" plants may include, but are not limited to, for example, almond, apple, apricot, blackberry, blueberry, cherry, peach, plum, quince, raspberry, rose, and strawberry.

The "Rubiaceae" plants may include, but are not limited to, for example, coffee.

The "Solanaceae" plants may include, but are not limited to, for example, bell pepper, chili pepper, eggplant, petunia, potato, tobacco, and tomato.

The "Vitaceae" plants may include, but are not limited to, for example, grape.

In the present disclosure, the term "plant growth" refers to the process in which plant seeds germinate to develop roots, stems, leaves, and flowers, or subsequently and gradually increase in size, weight, and quantity, and the term "promoting plant growth" refers to promoting the germination of plant seeds or an increase in the size and/or weight of germinated plants.

According to one embodiment, the N484 CP02-1006 strain and/or the N485 CP02-1007 strain may have activities for enhancing plant stress resistance and/or promoting plant growth. Specifically, when cucumbers (*Cucumis sativus*) are grown under cold stress conditions, the fresh weight, leaf length, and leaf width of the cucumbers are all reduced, but when the cucumber is treated with the N484 CP02-1006 strain or the N485 CP02-1007 strain, the reduced fresh weight, leaf length, and leaf width of the cucumbers may all be restored. For example, the N484 CP02-1006 strain or the N485 CP02-1007 strain may increase the fresh weight, leaf length, and leaf width of cucumbers that were reduced by cold stress, by 5 to 30%, 5 to 20% and 2 to 10%, respectively. That is, the N484 CP02-1006 strain or the N485 CP02-1007 strain may enhance plant stress resistance and promote plant growth, and thus may be usefully utilized for the purpose of enhancing plant stress resistance and promoting plant growth.

According to another aspect of the present disclosure, there is provided a bacterial composition comprising the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P.

In one embodiment, the composition may further comprise a cryoprotectant.

The *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P may be prepared in the form of a lyophilized composition to preserve the activity of these microbial cells, and the composition may further comprise a cryoprotectant to prevent damage to the cells.

In one embodiment, the cryoprotectant may include one or more selected from the group consisting of glycerol, ethylene glycol, propylene glycol, trehalose, sucrose, glucose, maltodextrin, sorbitol, skim milk powder, and starch.

In one embodiment, the cryoprotectant may be present in an amount of 0.01 to 30% by weight based on the total composition, specifically 0.01 to 30% by weight, 0.01 to 25% by weight, 0.01 to 20% by weight, 0.01 to 15% by weight, 0.1 to 30% by weight, 0.1 to 25% by weight, 0.1 to 20% by weight, 0.1 to 15% by weight, 1 to 30% by weight, 1 to 25% by weight, 1 to 20% by weight, or 1 to 15% by weight based on the total composition.

In one embodiment, the strain may be included in an amount of 10⁵ CFU/mL or more based on the total composition, and specifically, may be included in an amount of 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 10⁵ CFU/mL to 1 x 10¹² CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, or 1 x 10¹⁰ CFU/mL to 1 x 10¹² CFU/mL.

In one embodiment, the bacterial composition may be in a lyophilized form.

In one embodiment, the bacterial composition may be in the form of a lyophilized powder.

Another aspect of the present disclosure provides a composition for enhancing plant stress resistance.

The composition for enhancing plant stress resistance of the present disclosure comprises, as an active ingredient, one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

In the present disclosure, the term "culture" refers to a product obtained by culturing the strain, and the culture may encompass the culture broth itself in which the strain has been cultured, a culture filtrate obtained by removing the strain through filtration or centrifugation of the culture broth, and a supernatant obtained by centrifuging the culture broth or culture filtrate.

In the present disclosure, the term "concentrate" refers to a product obtained by concentrating the culture, and the term "extract" refers to a product obtained by extracting the culture using an appropriate solvent according to a known method. The term "dried product" refers to a product obtained by removing moisture from the culture or concentrate, and may be, but is not limited to, for example, a dried cell form of the strain.

In addition, the term "pulverized product" refers to a product obtained by pulverizing a dried product from which moisture has been removed from the cells or culture of the strain, and may be, but is not limited to, for example, a dried cell powder of the strain.

The culture of the strain may be prepared by inoculating the strain into a culture medium and culturing it according to a culture method known in the art, and the concentrate, extract, dried product or pulverized product may be prepared according to methods for treating or drying the strain or its culture known in the art.

In one embodiment of the composition for enhancing plant stress resistance, the stress may be cold stress.

In one embodiment of the composition for enhancing plant stress resistance, the plant may be one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

According to one embodiment, one or more selected from the group consisting of the *Bacillus amyloliquefaciens* N484 CP02-1006 strain deposited under Accession No. KCCM13395P, a culture of the strain, a concentrate of the culture, an extract of the culture, a dried product of the culture, and a pulverized product of the dried product may effectively increase the fresh weight, leaf length, and leaf width of plants reduced by cold stress, and thus may be usefully utilized for the purpose of enhancing plant stress resistance.

In addition, according to one embodiment, one or more selected from the group consisting of the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product may effectively mitigate the reduction in fresh weight, leaf length and leaf width of plants caused by cold stress, and thus may be usefully utilized for the purpose of enhancing plant stress resistance.

In one embodiment, the concentration of the *Bacillus amyloliquefaciens* N484 CP02-1006 strain deposited under Accession No. KCCM13395P in the composition for enhancing plant stress resistance may be 1 x 10⁵ CFU/mL to 1 x 10¹² CFU/mL. For example, the concentration of the strain may be 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, 1 x 10¹⁰ CFU/mL to 1 x 10¹² CFU/mL, or 1 x 10¹¹ CFU/mL to 1 x 10¹² CFU/mL.

In addition, in one embodiment, the concentration of the *Bacillus amyloliquefaciens* N485 CP02-1007 strain deposited under Accession No. KCCM13396P in the composition for enhancing plant stress resistance may be 1 x 10⁵ CFU/mL to 1 x 10¹² CFU/mL. For example, the concentration of the strain may be 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, 1 x 10¹⁰ CFU/mL to 1 x 10¹² CFU/mL, or 1 x 10¹¹ CFU/mL to 1 x 10¹² CFU/mL.

In one embodiment, when the composition for enhancing plant stress resistance is in a solid state rather than a liquid state, the unit "CFU/mL" used to describe the concentration of the strain may be changed to "CFU/g".

In the present disclosure, the composition for enhancing plant stress resistance may be prepared in the form of a directly sprayable solution, in the form of powder and suspension, or as a highly concentrated aqueous, oily or other suspension, dispersion, emulsion, oil dispersion, paste, dust, scatterable material, or granule, but is not limited thereto.

In the present disclosure, the composition for enhancing plant stress resistance may be formulated in various forms. The formulations may be prepared, for example, by adding solvents and/or carriers, and inert additives and surface-active substances, for example, emulsifiers or dispersants, may be mixed into the formulations. Suitable surface-active substances may be, but are not limited to, aromatic sulfonic acids (e.g. lignosulfonic acid, phenolsulfonic acid, naphthalene- and dibutylnaphthalenesulfonic acid), fatty acids, alkyl- and alkylarylsulfonates, alkyl lauryl ethers, alkali metal, alkaline earth metal and ammonium salts of fatty alcohol sulfates, sulfated hexa-, hepta- and octadecanol, salts of fatty alcohol glycol ethers, sulfonated naphthalene and derivatives thereof, condensates of formaldehyde, condensates of naphthalene or naphthalenesulfonic acid, phenol and formaldehyde, polyoxyethyleneoctyl phenol ethers, ethoxylated isooctyl-, octyl- or nonylphenols, alkylphenyl or tributylphenyl polyglycol ethers, alkylarylpolyether alcohols, isotridecyl alcohol, fatty alcohol/ethylene oxide condensates, ethoxylated castor oil, polyoxyethylene alkyl ether or polyoxypropylene, lauryl alcohol polyglycol ether acetate, sorbitol ester, lignin-sulfite waste liquor, or methylcellulose.

The carriers may be, but are not limited to, an agriculturally acceptable carrier, such as mineral earths (e.g., silica, silica gel, silicate, talc, kaolin, limestone, lime, chalk, boll, loess, clay, dolomite, diatomaceous earth, calcium sulfate, magnesium sulfate, magnesium oxide, ground synthetic materials), fertilizers (e.g., ammonium sulfate, ammonium phosphate, ammonium nitrate, urea), vegetable products (e.g., cereal flour, tree bark flour, wood meal, and nut shell flour), or cellulose powder, and the carriers may be used alone or in combination of two or more thereof.

In the present disclosure, the composition for enhancing plant stress resistance may further comprise any other component used in the art to enhance plant stress resistance. For example, it may further comprise any one or more selected from the group consisting of a plant growth regulator, a plant growth stimulant, and a plant nutrient.

The plant growth regulator, plant growth stimulant or plant nutrient may be a chemical or another bacterial strain, and may be a herbicide, bactericide, insecticide, fertilizer, or any combination thereof.

In the present disclosure, the composition for enhancing plant stress resistance may further comprise any one or more selected from the group consisting of other beneficial microorganisms, microbial active substances, organic fertilizers, and chemical fertilizers.

Other beneficial microorganisms may include *Bacillus spp.*, *Azotobacter spp.*, *Trichoderma spp.*, and *Saccharomyces spp.*, which are known to enhance plant stress resistance.

The microbial active substances may include enzyme precursors, microbial metabolites, organic acids, carbohydrates, enzymes, and trace elements. For example, the microbial active substances may include processed enzyme products (such as yeast autolysates), humic materials, seaweed extracts, starch, amino acids, and trace elements such as zinc, iron, copper, manganese, bromine, and molybdenum.

The chemical fertilizers may include various chemicals that can provide nitrogen, phosphorus and potassium nutrients to help enhance plant stress resistance. For example, the chemical fertilizers may include urea, calcium phosphate, potassium phosphate, and mixed nitrogen-phosphate-potassium (N-P-K) fertilizers, and may include other materials known in the art.

In the present disclosure, the composition for enhancing plant stress resistance may be used in combination with a spreading agent, a penetrating agent, or a surfactant to increase its absorption and effectiveness in plants.

In the present disclosure, the composition for enhancing plant stress resistance may further comprise preservatives, stabilizers, wetting agents, emulsifiers, cryoprotectants, or excipients.

For example, the composition for enhancing plant stress resistance may comprise the preservatives, stabilizers, or excipients in an effective amount sufficient to reduce the quality deterioration of the composition, thereby allowing the N484 CP02-1006 strain and/or the N485 CP02-1007 strain to survive or remain active for a longer period of time in the composition, unlike the N484 CP02-1006 strain and/or the N485 CP02-1007 strain that exist in nature.

The preservatives, stabilizers, or excipients may include, without limitation, those commonly used in the art as long as they can reduce the quality deterioration of the composition for enhancing plant stress resistance. For example, the stabilizers may be any one or more selected from the group consisting of alginate, sodium alginate, casein, sodium caseinate, cellulose, methylcellulose, carboxymethylcellulose, sodium carboxymethylcellulose, pectin, gelatin, gum arabic, xanthan gum, dextran, cyclodextrin, and starch. The excipients may include pharmaceutically acceptable solid carriers, liquid carriers, liquid diluents, liquefied gaseous diluents, solid diluents, or other adjuvants such as surfactants, for example, emulsifiers, dispersants or foaming agents.

The cryoprotectants may be included in the composition in an effective amount sufficient to reduce the quality deterioration of the composition when the composition for enhancing plant stress resistance is in a lyophilized state. For example, unlike the N484 CP02-1006 strain and/or the N485 CP02-1007 strain that exist in nature, the composition may comprise the cryoprotectants in an effective amount sufficient to reduce the quality deterioration of the composition in a lyophilized state, thereby allowing the N484 CP02-1006 strain and/or the N485 CP02-1007 strain to survive or remain active for a longer period of time within the lyophilized composition.

The cryoprotectants may include, without limitation, those commonly used in the art as long as they can reduce the quality deterioration of the composition in a lyophilized state. For example, the cryoprotectants may be any one or more selected from the group consisting of glycerol, ethylene glycol, propylene glycol, dimethyl sulfoxide (DMSO), glucose, trehalose, maltodextrin, dextrin, skim milk powder, and starch.

In one embodiment, the composition for enhancing plant stress resistance may be in the form of a lyophilized powder.

In one embodiment, the composition for enhancing plant stress resistance may be added to a plant fertilizer by drenching, spraying, or mixing.

The plant fertilizer may be prepared by a preparation method commonly used in the art. For example, the plant fertilizer may further comprise excipients or carriers commonly used in the art, and may be formulated into conventional plant fertilizer formulations known in the art, such as wettable powders, granules, dusts, emulsifiables, sprays, smokes, capsules, or gels.

In one embodiment, the plant fertilizer may be an environmentally friendly fertilizer capable of replacing chemical fertilizers, and the plant fertilizer may be used to enhance plant stress resistance.

Another aspect of the present disclosure provides a method for enhancing plant stress resistance.

The method for enhancing plant stress resistance of the present disclosure comprises treating a plant, an area surrounding the plant, or both, with one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

In the present disclosure, the plant may include any of roots, stems, leaves, seeds, fruits, or parts or combinations thereof, without limitation to the part and type of a plant. Specifically, the plant may be, but is not limited to, a plant individual capable of growing under stress conditions or one that has been grown under stress conditions.

In the present disclosure, the term "area surrounding the plant" refers to the range in which the active ingredient of the present disclosure may exert its effect, and includes both above-ground and underground areas. For example, it may be an area within about 2 m, within about 1 m, within about 70 cm, within about 50 cm, within about 25 cm, within about 10 cm, or within about 5 cm around a plant, a part of the plant, or fruit.

According to one embodiment, the method for enhancing plant stress resistance may effectively mitigate the reduction in fresh weight, leaf length, and leaf width of plants caused by cold stress, and thus may effectively enhance plant stress resistance.

Treatment of a plant with one or more selected from the group consisting of the strain, the culture of the strain, the concentrate of the culture, the extract of the culture, the dried product of the culture, and the pulverized product of the dried product may be performed by methods such as dipping, drenching, spraying, injection, infusion, mixing, or soil incorporation, but is not limited thereto as long as they can come into direct or indirect contact with the inside or outside of the plant. In addition, the treatment may be performed simultaneously with or separately from the growth of the plant under stress conditions, but is not limited thereto. For example, the treatment may be performed once or two or more times, but is not limited thereto. In addition, in the case of two or more treatments, the treatments may be performed at intervals of 3 to 15 days, 3 to 10 days, 3 to 7 days, 4 to 7 days, 5 to 7 days, or 6 days, but are not limited thereto.

Another aspect of the present disclosure provides the use of a composition comprising one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product in enhancing plant stress resistance.

Another aspect of the present disclosure provides a composition for promoting plant growth.

The composition for promoting plant growth of the present disclosure comprises, as an active ingredient, one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

In one embodiment of the composition for promoting plant growth, the plant may be one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

According to one embodiment, one or more selected from the group consisting of the *Bacillus amyloliquefaciens* N484 CP02-1006 strain deposited under Accession No. KCCM13395P, a culture of the strain, a concentrate of the culture, an extract of the culture, a dried product of the culture, and a pulverized product of the dried product may effectively increase the fresh weight, leaf length, and leaf width of plants reduced by cold stress, and thus may be usefully utilized for the purpose of promoting plant growth.

In addition, according to one embodiment, one or more selected from the group consisting of the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product may effectively increase the fresh weight, leaf length, and leaf width of plants reduced by cold stress, and thus may be usefully utilized for the purpose of promoting plant growth.

In one embodiment, the concentration of the *Bacillus amyloliquefaciens* N484 CP02-1006 strain deposited under Accession No. KCCM13395P in the composition for promoting plant growth may be 1 x 10⁵ CFU/mL to 1 x 10¹² CFU/mL. For example, the concentration of the strain may be 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, 1 x 10¹⁰ CFU/mL to 1 x 10¹² CFU/mL, or 1 x 10¹¹ CFU/mL to 1 x 10¹² CFU/mL.

In addition, in one embodiment, the concentration of the *Bacillus amyloliquefaciens* N485 CP02-1007 strain deposited under Accession No. KCCM13396P in the composition for promoting plant growth may be 1 x 10⁵ CFU/mL to 1 x 10¹² CFU/mL. For example, the concentration of the strain may be 1 x 10⁵ CFU/mL to 1 x 10⁶ CFU/mL, 1 x 10⁵ CFU/mL to 1 x 10⁷ CFU/mL, 1 x 10⁵ CFU/mL to 1 x 10⁸ CFU/mL, 1 x 10⁵ CFU/mL to 1 x 10⁹ CFU/mL, 1 x 10⁵ CFU/mL to 1 x 10¹⁰ CFU/mL, 1 x 10⁵ CFU/mL to 1 x 10¹¹ CFU/mL, 1 x 10⁶ CFU/mL to 1 x 10⁷ CFU/mL, 1 x 10⁶ CFU/mL to 1 x 10⁸ CFU/mL, 1 x 10⁶ CFU/mL to 1 x 10⁹ CFU/mL, 1 x 10⁶ CFU/mL to 1 x 10¹⁰ CFU/mL, 1 x 10⁶ CFU/mL to 1 x 10¹¹ CFU/mL, 1 x 10⁶ CFU/mL to 1 x 10¹² CFU/mL, 1 x 10⁷ CFU/mL to 1 x 10⁸ CFU/mL, 1 x 10⁷ CFU/mL to 1 x 10⁹ CFU/mL, 1 x 10⁷ CFU/mL to 1 x 10¹⁰ CFU/mL, 1 x 10⁷ CFU/mL to 1 x 10¹¹ CFU/mL, 1 x 10⁷ CFU/mL to 1 x 10¹² CFU/mL, 1 x 10⁸ CFU/mL to 1 x 10⁹ CFU/mL, 1 x 10⁸ CFU/mL to 1 x 10¹⁰ CFU/mL, 1 x 10⁸ CFU/mL to 1 x 10¹¹ CFU/mL, 1 x 10⁸ CFU/mL to 1 x 10¹² CFU/mL, 1 x 10⁹ CFU/mL to 1 x 10¹⁰ CFU/mL, 1 x 10⁹ CFU/mL to 1 x 10¹¹ CFU/mL, 1 x 10⁹ CFU/mL to 1 x 10¹² CFU/mL, 1 x 10¹⁰ CFU/mL to 1 x 10¹¹ CFU/mL, 1 x 10¹⁰ CFU/mL to 1 x 10¹² CFU/mL, or 1 x 10¹¹ CFU/mL to 1 x 10¹² CFU/mL.

In one embodiment, when the composition for promoting plant growth is in a solid state rather than a liquid state, the unit "CFU/mL" used to describe the concentration of the strain may be changed to "CFU/g".

In the present disclosure, the composition for promoting plant growth may be prepared in the form of a directly sprayable solution, in the form of powder and suspension, or as a highly concentrated aqueous, oily or other suspension, dispersion, emulsion, oil dispersion, paste, dust, scatterable material, or granule, but is not limited thereto, and may be formulated into various forms.

In the present disclosure, the composition for promoting plant growth may further comprise any other component used in the art to enhance plant stress resistance. For example, it may further comprise any one or more selected from the group consisting of a plant growth regulator, a plant growth stimulant, and a plant nutrient.

In the present disclosure, the composition for promoting plant growth may further comprise any one or more selected from the group consisting of other beneficial microorganisms, microbial active substances, organic fertilizers, and chemical fertilizers.

In the present disclosure, the composition for promoting plant growth may be used in combination with a spreading agent, a penetrating agent, or a surfactant to increase its absorption and effectiveness in plants.

In the present disclosure, the composition for promoting plant growth may further comprise preservatives, stabilizers, wetting agents, emulsifiers, cryoprotectants, or excipients.

In one embodiment, the composition for promoting plant growth may be added to a plant fertilizer by drenching, spraying, or mixing.

In one embodiment, the plant fertilizer may be an environmentally friendly fertilizer capable of replacing chemical fertilizers, and the plant fertilizer may be used to promote plant growth.

Another aspect of the present disclosure provides a method for promoting plant growth.

The method for promoting plant growth of the present disclosure comprises treating a plant, an area surrounding the plant, or both, with one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

In the present disclosure, the plant may include any of roots, stems, leaves, seeds, fruits, or parts or combinations thereof, without limitation to the part and type of a plant. Specifically, the plant may be, but is not limited to, a plant individual in need of growth promotion.

According to one embodiment, the method for promoting plant growth may effectively increase the reduced fresh weight, leaf length, and leaf width of plants, and thus may effectively promote plant growth.

Treatment of a plant with one or more selected from the group consisting of the strain, the culture of the strain, the concentrate of the culture, the extract of the culture, the dried product of the culture, and the pulverized product of the dried product may be performed by methods such as dipping, drenching, spraying, injection, infusion, mixing, or soil incorporation, but is not limited thereto as long as they can come into direct or indirect contact with the inside or outside of the plant. For example, the treatment may be performed once or two or more times, but is not limited thereto. In addition, in the case of two or more treatments, the treatments may be performed at intervals of 3 to 15 days, 3 to 10 days, 3 to 7 days, 4 to 7 days, 5 to 7 days, or 6 days, but are not limited thereto.

Another aspect of the present disclosure provides the use of a composition comprising one or more selected from the group consisting of the *Bacillus amyloliquefaciens* N484 CP02-1006 strain deposited under Accession No. KCCM13395P or the *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product in promoting plant growth.

### [Advantageous Effects]

The *Bacillus amyloliquefaciens* N484 CP02-1006 strain, the *Bacillus amyloliquefaciens* N485 CP02-1007 strain, or a culture thereof of the present disclosure may effectively restore the reduction in fresh weight, leaf length, and leaf width of plants caused by cold stress. Therefore, the strain or a culture thereof of the present disclosure can be usefully utilized as an environmentally friendly product that can promote plant production by enhancing cold stress resistance of plants.

However, the effects of the present disclosure are not limited to the effects mentioned above, and other effects that are not mentioned will be clearly understood by a person skilled in the art from the description below.

### [Description of Drawings]

FIG. 1 shows the results of measuring the fresh weight of cucumbers after treating them with a culture of *Bacillus amyloliquefaciens* N484 CP02-1006 strain or a culture of *Bacillus amyloliquefaciens* N485 CP02-1007 strain under cold stress conditions.
FIG. 2 shows the results of measuring the leaf length of cucumbers after treating them with a culture of *Bacillus amyloliquefaciens* N484 CP02-1006 strain or a culture of *Bacillus amyloliquefaciens* N485 CP02-1007 strain under cold stress conditions.
FIG. 3 shows the results of measuring the leaf width of cucumbers after treating them with a culture of *Bacillus amyloliquefaciens* N484 CP02-1006 strain or a culture of *Bacillus amyloliquefaciens* N485 CP02-1007 strain under cold stress conditions.

### [Best Mode]

Hereinafter, the present disclosure will be described in detail by examples. However, the following examples are only for specifically illustrating the present disclosure, and the content of the present disclosure is not limited by the following examples.

### Example 1. Isolation and culture of Bacillus amyloliquefaciens strains

The strains used in this experiment were obtained from field soil. Specifically, approximately 50 g of soil was collected at a depth of 5 to 10 cm from the ground surface, and then the collected soil was passed through a 2 mm mesh sieve to remove large stones and impurities. The filtered soil samples were divided into 5 g each and cultured in flasks, and the resulting culture was diluted 1:10 with sterilized distilled water, and then the microorganisms were purely isolated.

Among the isolated microorganisms, six strains showing high antagonistic activity against the representative plant pathogens *Rhizoctonia solani* (R.s), *Pythium ultimum* (P.u), and *Colletotrichum truncatum* (C.t) were selected. To confirm the bactericidal effects against plant pathogens, four paper discs were first placed on 8.5 cm diameter Petri dishes containing PDA (potato dextrose agarose) medium, and three of the paper discs were inoculated with the reference strain *Bacillus amyloliquefaciens* KACC 10116 strain, Serenade^{®} (Bayer, *B. velezensis* QST713 strain), and the strain to be evaluated, respectively. The remaining one paper disc was used as an untreated control. The three plant pathogens (R.s, P.u, C.t) were cultured on PDA (potato dextrose agarose) medium at 28 °C for 5 to 8 days, and then the pathogen colonies were placed in the center of the Petri dishes using a cork borer with a diameter of 5 mm. After culturing in an incubator at 25 °C for 7 to 20 days, the diameter of the zone of inhibition was examined to compare the antibacterial effects of the *Bacillus* strains. Finally, six strains showing antibacterial effects equal to or greater than those of Serenade^{®} were selected (Table 1).

**[Table 1]**

| **Antibacterial effect (unit: mm)** | | | |
|---|---|---|---|
| | *Rhizoctonia solani* | *Phythium ultium* | *Colletotrichum trucatum* |
| N311 | 9.7 | 3.7 | 12.7 |
| N474 | 10.3 | 3.3 | 11 |
| N481 | 9.3 | 5.0 | 12.3 |
| N484 | 8.7 | 1.0 | 11.7 |
| N485 | 4.3 | 4.7 | 6.7 |
| N490 | 9.7 | 4.0 | 10.7 |
| Serenade | 9.7 | 4.0 | 11.7 |

To identify the six strains selected as described above, nucleotide sequence analysis of the 16S rRNA gene was performed. Identification was performed by analyzing sequence identity with the previously reported *Bacillus amyloliquefaciens* reference strain KACC 10116 using the CLC Genomics Workbench 21 program (Qiagen). As a result of the analysis, each of the six strains was confirmed to have 99% sequence homology with the reference strain, and they were finally identified as *Bacillus amyloliquefaciens* strains: *Bacillus amyloliquefaciens* N474 CP02-1004 strain (Accession No. KCCM13393P), *Bacillus amyloliquefaciens* N481 CP02-1005 strain (Accession No. KCCM13394P), *Bacillus amyloliquefaciens* N484 CP02-1006 strain (Accession No. KCCM13395P), *Bacillus amyloliquefaciens* N485 CP02-1007 strain (Accession No. KCCM13396P), *Bacillus amyloliquefaciens* N490, *Bacillus amyloliquefaciens* N311. Each identified strain was suspended in 20% glycerol and stored.

Among the six finally selected strains, the N484 strain and the N485 strain were found to have the most excellent efficacy in mitigating cold stress according to subsequent experimental results. The N484 strain was named *Bacillus amyloliquefaciens* N484 CP02-1006 strain, was deposited internationally with the Korean Culture Center of Microorganisms, which is an international depository institution under the Budapest Treaty, and was assigned the Accession No. KCCM13395P on September 21, 2023. In addition, the N485 strain was named *Bacillus amyloliquefaciens* N485 CP02-1007 strain, was deposited internationally with the Korean Culture Center of Microorganisms, which is an international depository institution under the Budapest Treaty, and was assigned the Accession No. KCCM13396P on September 21, 2023.

The 16s rRNA gene nucleotide sequences of the N484 strain and the N485 strain are set forth in SEQ ID NO: 1 and SEQ ID NO: 2, respectively.

The six *Bacillus amyloliquefaciens* strains selected and identified as described above were cultured along with a *Bacillus amyloliquefaciens* strain isolated from Serenade (Bayer company) as a control. A singe colony of each *Bacillus amyloliquefaciens* strain was streaked onto LB (Luria-Bertani broth) medium and then primary cultured in an incubator at 28 °C for 20 hours. A colony was picked from the primary culture medium, streaked onto LB medium for a second time, and then subcultured in an incubator at 28 °C for 20 hours. Subsequently, 10 mL of sterilized distilled water was added to the secondary culture medium to perform cell re-suspension, and the cells were recovered using a spreader. The O.D (optical density) value of each strain was measured and then diluted to O.D = 1.0 for use in the experiment.

### Example 2. Evaluation of the effect of Bacillus amyloliquefaciens strains on mitigating plant cold stress.

An experiment was conducted to confirm the effect of the six *Bacillus amyloliquefaciens* strains cultured in Example 1 above on mitigating plant cold stress.

### 2-1. Experimental method

Cucumber (*Cucumis sativus*) was used as the plant, and cucumber seeds were sown in a 7:3 mixture of horticultural potting soil and vermiculite. Normal cucumber growth conditions were set as temperature 25°C, humidity 60 to 65%, and photoperiod 16h/8h (day/night), and cold stress growth conditions were set as temperature 10°C, humidity 60 to 65%, and photoperiod 16h/8h (day/night). Control groups were set as a DW-treated group under normal conditions, a DW-treated group under cold stress conditions, and a group treated with a culture of the *Bacillus amyloliquefaciens* strain isolated from Serenade (Bayer company), which is a commercial product control, under cold stress conditions. Test groups were set as groups treated with cultures of the six *Bacillus amyloliquefaciens* strains cultured in Example 1 above, under cold stress conditions.

Ten days after sowing cucumbers, the cultures (OD = 1.0, 1×10⁸ CFU/ml) of the *Bacillus amyloliquefaciens* strains cultured to an O.D = 1.0 value were foliar-applied at 5 mL/plant, twice at 7-day intervals. One day after the second application, the cucumbers were subjected to cold stress. After the cucumbers were grown for 7 days under cold stress conditions, the fresh weight, leaf length, and leaf width of the cucumbers were measured and compared. Specifically, the fresh weight was measured as the total weight of the above-ground parts of the cucumber, and the leaf length and leaf width were measured as the leaf length and leaf width of the largest leaf of each plant, respectively.

### 2-2. Measurement results of fresh weights

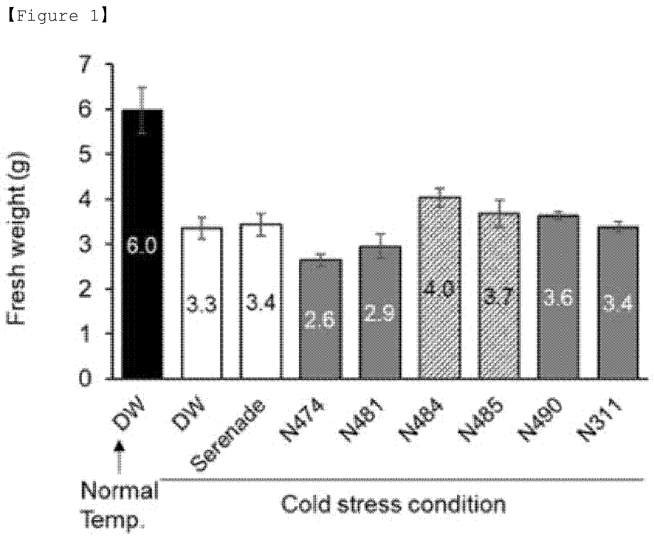

As a result of measuring fresh weight, when subject to cold stress, the fresh weight of cucumbers was remarkably reduced compared to normal conditions, but the reduction in fresh weight caused by cold stress was found to be restored in the *Bacillus amyloliquefaciens* N484 CP02-1006 strain-treated group and the N485 CP02-1007 strain-treated group (Fig. 1). Specifically, the N484 strain-treated group showed an increase in fresh weight of approximately 20.4% and 17.7% compared to the DW-treated group under cold stress conditions and the Serenade-treated group under cold stress conditions, respectively, and the N485 strain-treated group showed an increase in fresh weight of approximately 9.8% and 7.3% compared to the DW-treated group under cold stress conditions and the Serenade-treated group under cold stress conditions, respectively. In addition, the groups with the N474, N481, N490, and N311 strains showed fresh weights that were lower than or equal to those of the untreated group or the Serenade-treated group.

### 2-3. Measurement results of leaf lengths

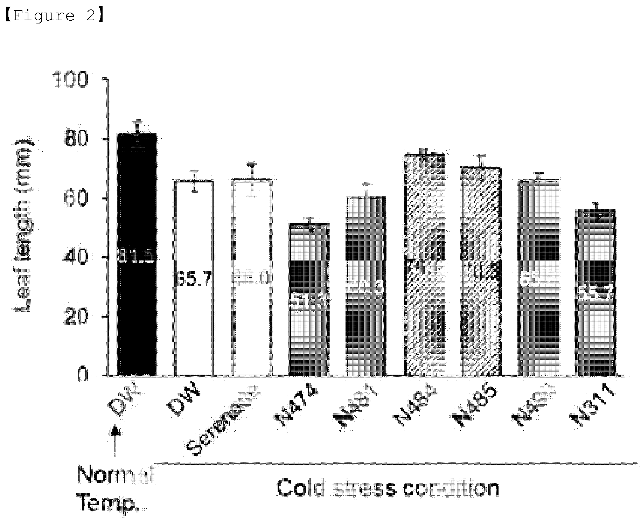

As a result of measuring leaf length, when subject to cold stress, the leaf length of cucumbers was remarkably reduced compared to normal conditions, but the reduction in leaf length caused by cold stress was found to be restored in the *Bacillus amyloliquefaciens* N484 strain-treated group and the N485 strain-treated group (FIG. 2). Specifically, the N484 strain-treated group showed an increase in leaf length of approximately 13.3% and 12.8% compared to the DW-treated group under cold stress conditions and the Serenade-treated group under cold stress conditions, respectively, and the N485 strain-treated group showed an increase in leaf length of approximately 6.9% and 6.4% compared to the DW-treated group under cold stress conditions and the Serenade-treated group under cold stress conditions, respectively. In addition, the N474, N481, N490, and N311 strain treatment groups showed leaf lengths that were lower than or equal to those of the untreated group or the Serenade treatment group.

### 2-4. Measurement results of leaf widths

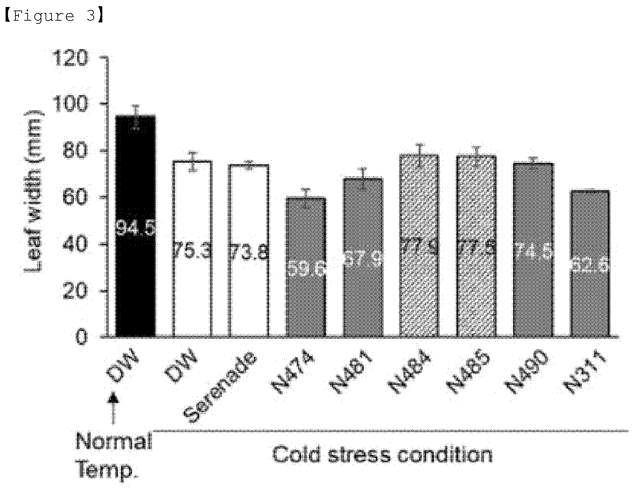

As a result of measuring leaf width, when subject to cold stress, the leaf width of cucumbers was remarkably reduced compared to normal conditions, but the reduction in leaf width caused by cold stress was found to be restored in the *Bacillus amyloliquefaciens* N484 strain-treated group and the N485 strain-treated group (FIG. 3). Specifically, the N484 strain-treated group showed an increase in leaf width of approximately 3.5% and 5.6% compared to the DW-treated group under cold stress conditions and the Serenade-treated group under cold stress conditions, respectively, and the N485 strain-treated group showed an increase in leaf width of approximately 3.0% and 5.1% compared to the DW-treated group under cold stress conditions and the Serenade-treated group under cold stress conditions, respectively. In addition, the N474, N481, N490, and N311 strain treatment groups showed leaf widths that were lower than or equal to those of the untreated group or the Serenade treatment group.

Through the above experiments, it was confirmed that the *Bacillus amyloliquefaciens* N484 CP02-1006 strain and N485 CP02-1007 strain could effectively mitigate cold stress by promoting plant growth when plants were subjected to cold stress. Therefore, it is expected that these strains can be usefully utilized for the purpose of enhancing plant stress resistance and promoting plant growth.

Although representative embodiments of the present disclosure have been described above for illustrative purposes, the scope of the present disclosure is not limited only to the specific embodiments described above, and appropriate changes will be able to be made by a person skilled in the art within the scope described in the claims of the present disclosure.

### [Accession No.]

Name of depository institution: Korean Culture Center of Microorganisms
Accession No.: KCCM13395P
Accession date: 20230921

Name of depository institution: Korean Culture Center of Microorganisms
Accession No.: KCCM13396P
Accession date: 20230921

### [Original text of the deposit certificate]

Translation

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the DEPOSITOR:
Bacillus amyloliquefaciens N484 CP02-1006 | Accession number given by the INTERNATIONAL DEPOSITARY AUTHORITY:
KCCM13395P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 21. 2023 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenee-2ga-gil
Seodaemun-gu
SEOUL 03641
Republic of Korea | Signature(s) of person(s) having the power
to represent the International Depositary
Authority or of authorized official(s)

Date: September. 21, 2023

한국미생물보존센터

¹ Where Rule 6.4(d) applies, such date is the date on which the status of international depositary authority was acquired.

Form BP/4 (sole page)

한국미생물보존센터 [?]
02611 [?] Tel: 02-391-2950 390-3950 Fax: 02-392-2859
KOREAN CULTURE CENTER OF MICROORGANISMS [?]
Yurim Blog. 45, Hongjenen 2ga gil, Seodaemun gu, Seoul, 03641, Korea Tel. 82-2-031-0950 398-0959 Fax: 82-2-391-2959

Translation

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Bacillus amyloliquefaciens N484 CP02-1007 | Accession number given by the
INTERNATIONAL DEPOSITARY AUTHORITY:
KCCM13396P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 21. 2023 (date of the original deposit)¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 21. 2023

¹ Where Rule 6.4(d) applies, such date is the date on which the status of international depositary authority was
acquired.

Form BP/4 (sole page)

한국미생물보존센터 [?]
03641 [?] Tel 02-391-0950, 396-0950 Fax 02-392-2859
KOREAN CULTURE CENTER OF MICROORGANISMS [?]
Yurim B/D 45 Hongjenae-2ga-gil Seodaemun-gu Seoul 03641 Korea Tel: 82-2-391-0950, 396-0950 Fax: 82-2-392-2859

## Claims

1. A *Bacillus amyloliquefaciens* N484 CP02-1006 strain deposited under Accession No. KCCM13395P, or a *Bacillus amyloliquefaciens* N485 CP02-1007 strain deposited under Accession No. KCCM13396P.

2. The strain according to claim 1, wherein the strain enhances plant stress resistance.

3. The strain according to claim 2, wherein the stress is cold stress.

4. The strain according to claim 1, wherein the strain promotes plant growth.

5. A composition for enhancing plant stress resistance, comprising: as an active ingredient, one or more selected from the group consisting of a *Bacillus amyloliquefaciens* N484 CP02-1006 strain deposited under Accession No. KCCM13395P or a *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

6. The composition for enhancing plant stress resistance according to claim 5, wherein the stress is cold stress.

7. The composition for enhancing plant stress resistance according to claim 5, wherein the plant is one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

8. A composition for promoting plant growth, comprising, as an active ingredient, one or more selected from the group consisting of a *Bacillus amyloliquefaciens* N484 CP02-1006 strain deposited under Accession No. KCCM13395P or a *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

9. The composition for promoting plant growth according to claim 8, wherein the plant is one or more selected from the group consisting of Cucurbitaceae, Brassicaceae, Amaranthaceae, Amaryllidaceae, Apiaceae, Asparagaceae, Asteraceae, Bromeliaceae, Convolvulaceae, Fabaceae, Malvaceae, Musaceae, Poaceae, Polygonaceae, Rosaceae, Rubiaceae, Solanaceae, and Vitaceae.

10. A method for enhancing plant stress resistance, comprising treating a plant, an area surrounding the plant, or both, with one or more selected from the group consisting of a *Bacillus amyloliquefaciens* N484 CP02-1006 strain deposited under Accession No. KCCM13395P or a *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.

11. A method for promoting plant growth, comprising treating a plant, an area surrounding the plant, or both, with one or more selected from the group consisting of a *Bacillus amyloliquefaciens* N484 CP02-1006 strain deposited under Accession No. KCCM13395P or a *Bacillus amyloliquefaciens* N485 CP02-1007 strain deposited under Accession No. KCCM13396P, a culture of the strain, a concentrate of the culture, an extract of the culture, a dried product of the culture, and a pulverized product of the dried product.
